(19) Europäisches Patentamt
European Patent Office
Office européen des brevets

(11) Veröffentlichungsnummer: **0 515 901 A1**

(12) **EUROPÄISCHE PATENTANMELDUNG**

(21) Anmeldenummer: **92108035.4**

(22) Anmeldetag: **13.05.92**

(51) Int. Cl.5: **A61K 31/00**, A61K 31/165, A61K 31/33, A61K 31/215

(30) Priorität: **28.05.91 DE 4117371**

(43) Veröffentlichungstag der Anmeldung: **02.12.92 Patentblatt 92/49**

(84) Benannte Vertragsstaaten: **AT BE CH DE DK ES FR GB IT LI NL PT SE**

(71) Anmelder: **BASF Aktiengesellschaft**
**Carl-Bosch-Strasse 38**
**W-6700 Ludwigshafen(DE)**

(72) Erfinder: **Sauter, Hubert, Dr.**
**Neckarpromenade 20**
**W-6800 Mannheim 1(DE)**
Erfinder: **Lorenz, Gisela, Dr.**
**Erlenweg 13**
**W-6730 Neustadt(DE)**
Erfinder: **Steiner, Gerd, Dr.**
**Oberer Waldweg 1**
**W-6719 Kirchheim(DE)**
Erfinder: **Janssen, Bernd, Dr.**
**Leuschnerstrasse 18a**
**W-6700 Ludwigshafen(DE)**
Erfinder: **Anke, Timm, Prof. Dr.**
**Theodor-Heuss-Strasse 17**
**W-6750 Kaiserslautern(DE)**
Erfinder: **Steglich, Wolfgang, Prof. Dr.**
**Goerresstrasse 8**
**W-8000 Muenchen 40(DE)**

(54) **Antimykotische Mittel, die Phenylessigsäurederivate enthalten.**

(57) Antimykotische Mittel enthaltend eine Verbindung der Formel

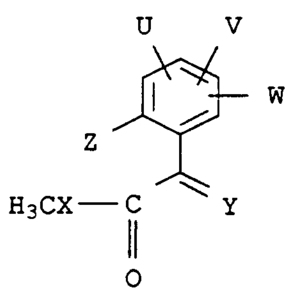

1

in der

=Y $=CH-OCH_3$, $=CH-CH_3$, $=CH-CH_2-CH_3$, $=CH-SCH_3$ oder $=N-OCH_3$ und X Sauerstoff bedeutet, oder in der X auch NH bedeuten kann, wenn Y = $N-OCH_3$ ist,

Z Halogen, Nitro, Cyano, gegebenenfalls substituiertes Alkyl, gegebenenfalls substituiertes Cycloalkyl, gegebenenfalls substituiertes Aralkyl, gegebenenfalls substituiertes Aryloxyalkyl, gegebenenfalls substituiertes Arylthioalkyl, gegebenenfalls substituiertes Heteroarylalkyl, gegebenenfalls substituiertes Heteroaryloxyalkyl, gegebenenfalls substituiertes Heteroarylthioalkyl, gegebenenfalls substituiertes Alkenyl, gegebenenfalls substituiertes Aralkenyl, gegebenenfalls substituiertes Aryloxyalkenyl, gegebenenfalls substituiertes Arylthioalkenyl, gegebenenfalls substituiertes Heteroarylalkenyl, gegebenenfalls substituiertes Heteroaryloxyalkenyl, gegebenenfalls substituiertes Heteroarylthioalkenyl, gegebenenfalls substituiertes Alkinyl, gegebenenfalls substituiertes Aryl, gegebenenfalls substituiertes Heteroaryl, gegebenenfalls substituiertes Amino, gegebenenfalls substitu-

EP 0 515 901 A1

iertes Arylazo, gegebenenfalls substituiertes Acylamino, $OR^{12}$, $SR^{13}$, $SOR^{14}$, $SO_2R^{15}$, $-COOR^{16}$, $-CONR^{17}R^{18}$, $-COR^{19}$, $-CR^{20}=NR^{21}$, $-N=CR^{22}R^{23}$, $-CR^{24}=N-OR^{25}$, $-CR^{25}R^{26}-O-N=CR^{27}R^{28}$ bedeutet und

U, V, W Wasserstoff bedeuten oder eine der für Z genannten Bedeutungen haben können,
oder in der
zwei der Gruppierungen Z, U, V oder W in benachbarten Positionen des Phenylrings gegebenenfalls zusammen einen gegebenenfalls substituierten, an den Phenylring ankondensierten fünf- oder sechsgliedrigen, aromatischen oder aliphatischen Ring bilden, der ggf. ein bis drei Heteroatome (N, S, O) enthalten kann,
wobei die Gruppierungen $R^{12}$ bis $R^{28}$ gegebenenfalls gleich oder verschieden Wasserstoff, gegebenenfalls substituiertes $C_1$-$C_8$-Alkyl, gegebenenfalls substituiertes $C_2$-$C_8$-Alkenyl, gegebenenfalls substituiertes $C_2$-$C_8$-Alkinyl, gegebenenfalls substituiertes Cycloalkyl, gegebenenfalls substituiertes Cycloalkylalkyl, gegebenenfalls substituiertes Aryl, gegebenenfalls substituiertes Heteroaryl, gegebenenfalls substituiertes Aralkyl, gegebenenfalls substituiertes Heteroarylalkyl, gegebenenfalls substituiertes Aryloxyalkyl, gegebenenfalls substituiertes Arylthioalkyl, gegebenenfalls substituiertes Heteroaryloxyalkyl oder gegebenenfalls substituiertes Heteroarylthioalkyl bedeuten,

und ihre Anwendung zur Bekämpfung von Mykosen.

Die vorliegende Erfindung betrifft antimykotische Mittel, die Phenylessigsäurederivate enthalten, und die Verwendung dieser Derivate als Antimykotika.

Es ist bekannt, Phenylessigsäurederivate als Fungizide im Pflanzenschutz zu verwenden (EP 178 826, 203 606, 203 608, 226 917, 229 974, 242 070, 242 081, 244 077, 251 082, 253 213, 254 426, 256 667, 260 794, 267 734, 270 252, 278 595, 280 185, 291 196, 299 694, 307 103, 310 954, 336 211, 337 211, 341 845, 342 459, 350 691, 354 571, 363 818, 370 629, 374 811, 378 308, 378 755, 379 o98, 382 375, 385 224, 385 357, 386 561, 393 428, 393 861, 398 692, 400 417, 405 782, 422 597, 426 460, 459 285, 460 575, 463 488, 468 684, 468 695, 468 775). Ein Hinweis auf antimykotische Wirkung findet sich dort nicht.

Es wurde nun gefunden, daß Verbindungen der Formel

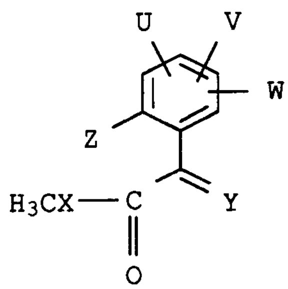

1

in der

=Y   $=CH\text{-}OCH_3$, $=CH\text{-}CH_3$, $=CH\text{-}CH_2\text{-}CH_3$, $=CH\text{-}SCH_3$ oder $=N\text{-}OCH_3$ bedeutet,

X   ein Sauerstoffatom oder - falls Y eine $=N\text{-}OCH_3$-Gruppe ist - auch eine NH-Gruppe bedeutet,

Z   Halogen (F, Cl, Br, J), Nitro, Cyano, gegebenenfalls substituiertes Alkyl, gegebenenfalls substituiertes Cycloalkyl, gegebenenfalls substituiertes Aralkyl, gegebenenfalls substituiertes Aryloxyalkyl, gegebenenfalls substituiertes Arylthioalkyl, gegebenenfalls substituiertes Heteroarylalkyl, gegebenenfalls substituiertes Heteroaryloxyalkyl, gegebenenfalls substituiertes Heteroarylthioalkyl, gegebenenfalls substituiertes Heteroarylthioalkyl, gegebenenfalls substituiertes Alkenyl, gegebenenfalls substituiertes Aralkenyl, gegebenenfalls substituiertes Aryloxyalkenyl, gegebenenfalls substituiertes Arylthioalkenyl, gegebenenfalls substituiertes Heteroarylalkenyl, gegebenenfalls substituiertes Heteroaryloxyalkenyl, gegebenenfalls substituiertes Heteroarylthioalkenyl, gegebenenfalls substituiertes Alkinyl, gegebenenfalls substituiertes Aryl, gegebenenfalls substituiertes Heteroaryl, gegebenenfalls substituiertes Amino, gegebenenfalls substituiertes Arylazo, gegebenenfalls substituiertes Acylamino, $OR^{12}$, $SR^{13}$, $SOR^{14}$, $SO_2R^{15}$, $-COOR^{16}$, $-CONR^{17}R^{18}$, $-COR^{19}$, $-CR^{20}=NR^{21}$, $-N=CR^{22}R^{23}$, $-CR^{24}=N\text{-}OR^{25}$, $-CR^{25}R^{26}\text{-}O\text{-}N=CR^{27}R^{28}$ bedeutet und

U, V, W gegebenenfalls gleich oder verschieden sind und Wasserstoff bedeuten oder eine der für Z genannten Bedeutungen haben können,

oder in der

zwei der Gruppierungen Z, U, V oder W in benachbarten Positionen des Phenylrings gegebenenfalls zusammen einen gegebenenfalls substituierten, an den Phenylring ankondensierten fünf- oder sechsgliedrigen, aromatischen oder aliphatischen Ring bilden, der ggf. ein bis drei Heteroatome (N, S, O) enthalten kann, und

die Gruppierungen $R^{12}$ bis $R^{28}$ gegebenenfalls gleich oder verschieden sind und Wasserstoff, gegebenenfalls substituiertes $C_1$-$C_8$-Alkyl, gegebenenfalls substituiertes $C_2$-$C_8$-Alkenyl, gegebenenfalls substituiertes $C_2$-$C_8$-Alkinyl, gegebenenfalls substituiertes Cycloalkyl, gegebenenfalls substituiertes Cycloalkylalkyl, gegebenenfalls substituiertes Aryl, gegebenenfalls substituiertes Heteroaryl, gegebenenfalls substituiertes Aralkyl, gegebenenfalls substituiertes Heteroarylalkyl, gegebenenfalls substituiertes Aryloxyalkyl, gegebenenfalls substituiertes Arylthioalkyl, gegebenenfalls substituiertes Heteroaryloxyalkyl oder gegebenenfalls substituiertes Heteroarylthioalkyl bedeuten,

eine gute antimykotische Wirkung haben. Die Verbindungen und ihre Herstellung sind bekannt (siehe die eingangs zitierten europäischen Offenlegungsschriften).

Ein Bestandteil der Erfindung sind antimykotische Mittel, die Verbindungen der Formel 1 enthalten, in denen U, V und W gleich oder verschieden sind und Wasserstoff, Fluor, Chlor, Brom, Iod, Cyano, Nitro, Trifluormethyl, Methyl oder Methoxy bedeuten.

Ein weiterer Bestandteil der Erfindung sind antimykotische Mittel, die Verbindungen der Formel 1 enthalten, in denen U, V und W Wasserstoff bedeuten.

Ein weiterer Bestandteil der Erfindung sind antimykotische Mittel, die Verbindungen der Formel 1

enthalten, in denen Z für die Gruppe $OR^{12}$ oder $SR^{13}$ steht und $R^{12}$ und $R^{13}$ die oben angegebenen Bedeutungen haben.

Ein weiterer Bestandteil der Erfindung sind antimykotische Mittel, die Verbindungen der Formel 1 enthalten, in denen $R^{12}$ und $R^{13}$ gegebenenfalls substituiertes Alkyl, gegebenenfalls substituiertes Aryl, oder gegebenenfalls substituiertes fünf- oder sechsgliedriges Heteroaryl mit 1 bis 3 Heteroatomen (N, O, S) bedeuten.

Ein weiterer Bestandteil der Erfindung sind antimykotische Mittel, die Verbindungen der Formel 1a enthalten,

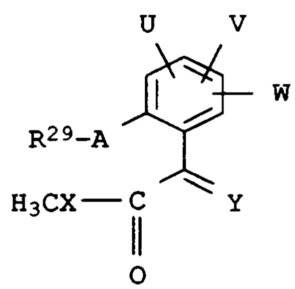

1a

in der U, V, W, X und Y die oben angegebene Bedeutung haben und -A- für die Gruppierungen $-CR^{30}=CR^{31}-$, $-CHR^{30}-CHR^{31}-$, $-O-CHR^{30}-$, $-CHR^{30}-O-$, $-CHR^{30}-S-$, $-S-CHR^{30}-$, $-O-N=CR^{30}-$ oder $-CHR^{30}-$ steht und in der $R^{29}$ gegebenenfalls substituiertes Alkyl, gegebenenfalls substituiertes Alkenyl, gegebenenfalls substituiertes Aryl, gegebenenfalls substituiertes Heteroaryl, gegebenenfalls substituiertes Aralkyl, gegebenenfalls substituiertes Heteroarylalkyl bedeuten und $R^{30}$ und $R^{31}$ gleich oder verschieden sind und Wasserstoff oder geradkettiges oder verzweigtes $C_1$-$C_4$-Alkyl bedeuten.

Ein weiterer Bestandteil der Erfindung sind antimykotische Mittel, die Verbindungen der Formel 1a enthalten, in denen die Gruppe $R^{29}$-A- für die Gruppierung $R^{32}R^{33}C=N-O-CHR^{31}-$ steht, in der $R^{31}$ die oben genannten Bedeutungen hat und $R^{32}$ und $R^{33}$ unabhängig voneinander Wasserstoff, $C_1$-$C_{12}$-Alkyl, $C_1$-$C_4$-Halogenalkyl, $C_1$-$C_4$-Alkoxy-$C_1$-$C_4$-alkyl, $C_1$-$C_4$-Alkylthio-$C_1$-$C_4$-alkyl, Aryl-$C_1$-$C_4$-alkyl, Aryloxy-$C_1$-$C_4$-alkyl, Arylthio-$C_1$-$C_4$-alkyl, Heteroaryl-$C_1$-$C_4$-alkyl, $C_2$-$C_{12}$-Alkenyl, Aryl-$C_2$-$C_4$-alkenyl bedeuten, Heteroaryl-$C_2$-$C_4$-alkenyl, $C_2$-$C_{12}$-Alkinyl, $C_3$-$C_6$-Cycloalkyl, Aryl, Heteroaryl, Cyano oder eine der Gruppen (a) bis (d)

$COOR^{34}$ (a) $CONR^{35}R^{36}$ (b)

$COR^{37}$ (c) $CR^{38}=NOR^{39}$ (d) bedeuten

oder $R^{32}$ und $R^{33}$ zusammen mit dem Kohlenstoffatom, an das sie gebunden sind, einen 4- bis 7-gliedrigen, gegebenenfalls ein Sauerstoff- oder Schwefelatom enthaltenden Ring, welch letzterer einen oder zwei ankondensierte aromatische Ringe, z.B. gegebenenfalls substituierte Benzolringe enthalten kann, bedeuten und $R^{34}$, $R^{35}$, $R^{36}$, $R^{37}$, $R^{38}$ und $R^{39}$ jeweils Wasserstoff, $C_1$-$C_4$-Alkyl, Aryl oder Heteroaryl bedeuten.

Ein weiterer Bestandteil der Erfindung sind antimykotische Mittel, die Verbindungen der Formel 1a enthalten, in denen $R^{29}$ für fünf- oder sechsgliedriges, gegebenenfalls ein- oder mehrfach durch Halogen, $C_1$-$C_{12}$-Alkyl, $C_1$-$C_{12}$-Halogenalkyl, $C_2$-$C_{12}$-Alkenyl, $C_2$-$C_{12}$-Alkinyl, gegebenenfalls substituiertes Aryl, gegebenenfalls substituiertes Heteroaryl, gegebenenfalls substituiertes Aralkyl, $C_1$-$C_{12}$-Alkoxy, $C_1$-$C_{12}$-Halogenalkoxy, $C_2$-$C_{12}$-Alkenyloxy, $C_2$-$C_{12}$-Alkinyloxy, gegebenenfalls substituiertes Aryloxy, Formyl, $C_1$-$C_{12}$-Acyl, Cyano, Trifluormethyl, Nitro oder mit der Gruppe $-CR^{34}=N-OR^{35}$ substituiertes und/oder gegebenenfalls mit einem Benzolring anelliertes Aryl, Benzyl oder Heteroaryl bedeutet, wobei $R^{34}$ für Wasserstoff oder $C_1$-$C_4$-Alkyl und $R^{35}$ für Wasserstoff, $C_1$-$C_8$-Alkyl, $C_2$-$C_8$-Alkenyl oder $C_2$-$C_8$-Alkinyl steht.

Zur Herstellung der Wirkstoffe wird ergänzend darauf verwiesen, daß die Verbindungen, in denen Y die verschiedenen oben genannten Bedeutungen hat, aus den Ketoestern der Formel 2

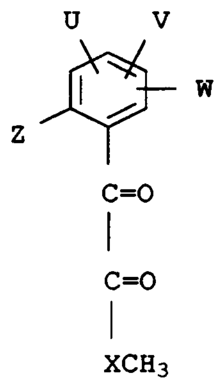

2

in der U, V, W, X und Z die oben genannten Bedeutungen haben, nach bekannten Verfahren hergestellt werden können.

Im übrigen wird die Herstellung der Ketoester 2 in den oben genannten Veröffentlichungen beschrieben.

Beispiele für Verbindungen der Formel I, die in den erfindungsgemäßen antimykotischen Mitteln enthalten sein können, sind in Tabelle 1 aufgeführt. Weitere Beispiele sind in den oben genannten Europäischen Patentanmeldungen angegeben.

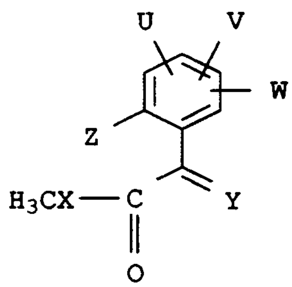

1

Die Verbindungen können bezüglich der C = Y-Doppelbindung als Isomere vorliegen. Bevorzugt sind die E-Isomeren.

Tabelle 1

| Nr. | Z- | U, V, W | =Y (Isomer) | X |
|---|---|---|---|---|
| 1 | 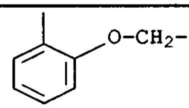 | H, H, H | $=CH-OCH_3$ (E) | O |
| 2 | 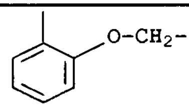 | H, H, H | $=N-OCH_3$ (E) | O |
| 3 | 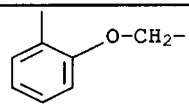 | H, H, H | $=CH-CH_3$ (E) | O |
| 4 | 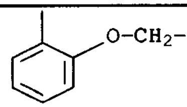 | H, H, H | $=CH-CH_2-CH_3$ (E) | O |
| 5 | 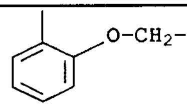 | H, H, H | $=CH-SCH_3$ (E) | O |
| 6 | 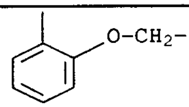 | H, H, H | $=N-OCH_3$ (E) | NH |
| 7 | 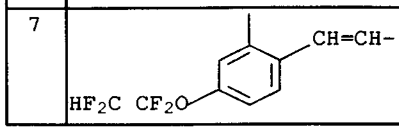 | H, H, H | $=CH-OCH_3$ (E) | |

| Nr. | Z- | U, V, W | =Y (Isomer) | X |
|---|---|---|---|---|
| 8 | 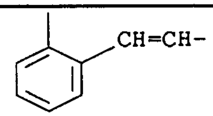 | H, H, H | $=N-OCH_3$ (E) | O |
| 9 | 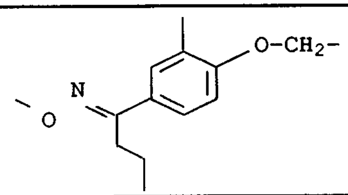 | H, H, H | $=N-OCH_3$ (E) | O |
| 10 | 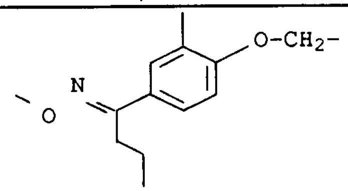 | H, H, H | $=CH-OCH_3$ (E) | O |
| 11 | 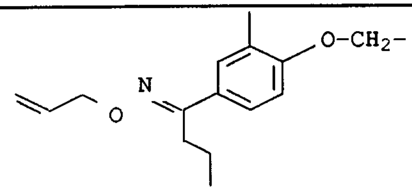 | H, H, H | $=N-OCH_3$ (E) | O |

| Nr. | Z- | U, V, W | =Y (Isomer) | X |
|---|---|---|---|---|
| 12 | 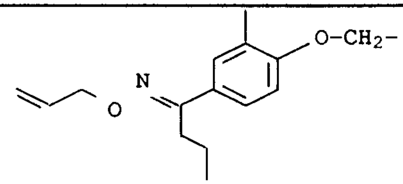 | H, H, H | $=CH-OCH_3$ (E) | O |
| 13 | 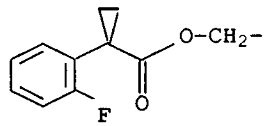 | H, H, H | $=CH-OCH_3$ (E) | O |
| 14 | 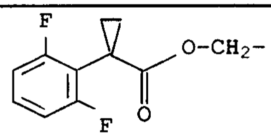 | H, H, H | $=CH-OCH_3$ (E) | O |
| 15 | 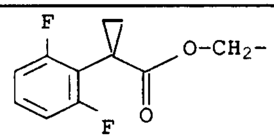 | H, H, H | $=N-OCH_3$ (E) | O |
| 16 | 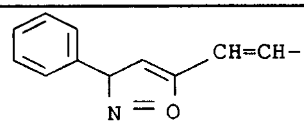 | H, H, H | $=CH-OCH_3$ (E) | O |
| 17 | 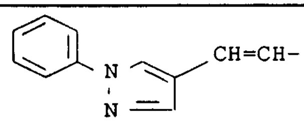 | H, H, H | $=CH-OCH_3$ (E) | O |

| Nr. | Z- | U, V, W | =Y (Isomer) | X |
|---|---|---|---|---|
| 18 | 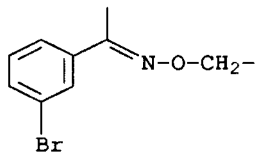 | H, H, H | $=N-OCH_3$ (E) | O |
| 19 | 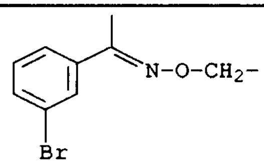 | H, H, H | $=CH-OCH_3$ (E) | O |
| 20 | 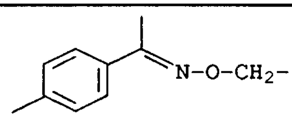 | H, H, H | $=N-OCH_3$ (E) | O |
| 21 | 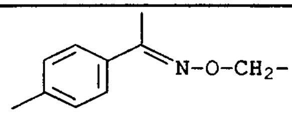 | H, H, H | $=N-OCH_3$ (E) | NH |
| 22 | 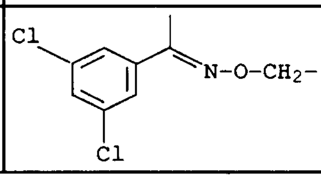 | H, H, H | $=N-OCH_3$ (E) | O |

| Nr. | Z- | U, V, W | =Y (Isomer) | X |
|---|---|---|---|---|
| 23 | 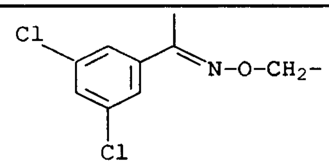 | H, H, H | $=N-OCH_3$ (E) | NH |
| 24 | 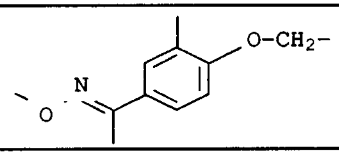 | H, H, H | $=CH-OCH_3$ (E) | O |
| 25 | 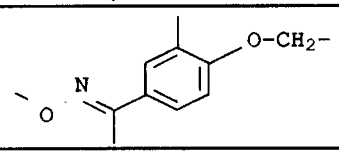 | H, H, H | $=CH-CH_3$ (E) | O |
| 26 | 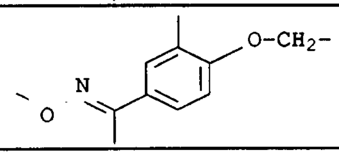 | H, H, H | $=CH-CH_2-CH_3$ (E) | O |
| 27 | 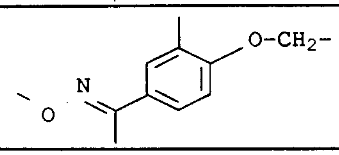 | H, H, H | $=N-OCH_3$ (E) | O |
| 28 | 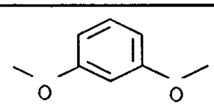 | H, H, H | $=CH-OCH_3$ (E) | O |

| Nr. | Z- | U, V, W | =Y (Isomer) | X |
|---|---|---|---|---|
| 29 | 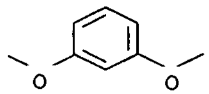 | H, H, H | $=N-OCH_3$ (E) | O |
| 30 | 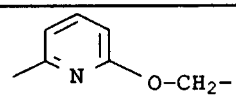 | H, H, H | $=CH-OCH_3$ (E) | O |
| 31 | 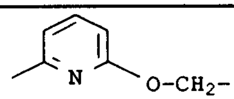 | H, H, H | $=N-OCH_3$ (E) | O |
| 32 | 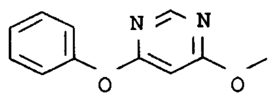 | H, H, H | $=CH-OCH_3$ (E) | O |
| 33 | 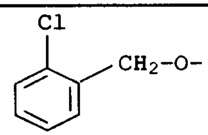 | H, H, H | $=N-OCH_3$ (E) | O |
| 34 | 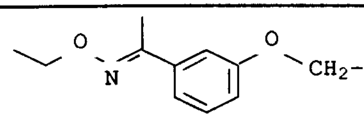 | H, H, H | $=N-OCH_3$ (E) | O |
| 35 | 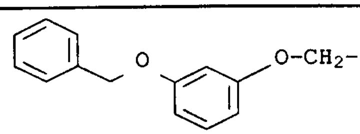 | H, H, H | $=N-OCH_3$ (E) | O |

| Nr. | Z- | U, V, W | =Y (Isomer) | X |
|---|---|---|---|---|
| 36 | 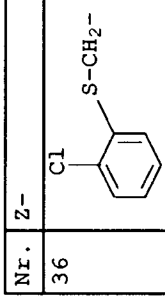 | H, H, H | $=CH-OCH_3$ (E) | O |
| 37 | 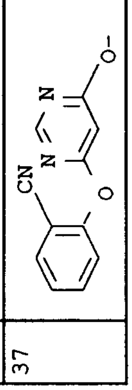 | H, H, H | $=N-OCH_3$ (E) | O |

Überraschenderweise zeigen die Phenylessigsäurederivate neben einer sehr guten antimykotischen In-vitro-Wirksamkeit eine gute, therapeutisch nutzbare In-vivo-Wirksamkeit, insbesondere gegen Dermatophyten, aber auch gegen andere Keime. Sie besitzen auch antibakterielle Wirksamkeiten. Die Wirkstoffe stellen somit eine wertvolle Bereicherung der Pharmazie dar.

Die Wirkung gegen Dermatophyten, Bakterien und Protozoen kann nach Methoden, wie sie beispielsweise in P. Klein, Bakteriologische Grundlagen der chemotherapeutischen Laboratoriumspraxis, Springer-Verlag, Berlin, 1957, beschrieben wird, aufgezeigt werden. Die Wirkung gegenüber Hefen kann im Pseudomycel- bzw. Mycelphasentest nachgewiesen werden (vgl. DE-OS 30 20 093).

Die Ermittlung der minimalen Hemmkonzentration (MHK) erfolgte im Agardilutionsverfahren gemaß DIN 58 940/ICS.

Hierzu wurden Petrischalen mit 9 cm Durchmesser mit 20 ml frisch hergestelltem und bei 50 °C flüssig gehaltenem Müller-Hinton-Agar (Fa. Merck, Art.Nr. 5337) unter sterilen Kautelen (LF) beschickt, dem die jeweilige Wirkstofflösung in 10 Vol.-% zugesetzt wurde. Die Proben sind in DMSO gut löslich. Jeweils 10,0 mg wurden in 10,0 ml DMSO gelöst und mit sterilem A.bidest. weiter verdünnt. Die nach dem Vermischen mit dem Testagar erzielten Endkonzentrationen sind der Ergebnistabelle 2 zu entnehmen. Platten mit der jeweils höchsten Lösungsmittelkonzentration (Kontrolle K 1) sowie mit 10 % A.bidest. (Kontrolle K 2) ohne Wirkstoff dienten der vergleichenden Wachstumskontrolle.

Nach Verfestigung und Trocknung (ca. 1 Std. bei 37 °C) wurden die Testplatten punktförmig mit jeweils 10 µl der Testkeimsuspension ("Inoculum") beimpft.

Bezüglich der Herstellung der Inocula wurde gemaß den NCCLS/FDA-Empfehlungen verfahren. Nach Anzucht auf festen Medien, Reinheits- und Identitätskontrolle wurden einige Kolonien in sterile Müller-Hinton-Bouillon (Fa. Merck, Art.Nr. 10293) überimpft und bis zur sichtbaren Trübung bebrütet. Diese Kulturen wurden durch Hinzufügen steriler Bouillon so verdünnt, daß sie der Trübung des McFarland-Standards 0,5 entsprachen (= ca. 10(8) KBE/ml). Eine weitere 1:10-Verdünnung diente als Inoculum, deren Keimdichte parallel nochmals per Spiralometer ermittelt wurde.

Die inokulierten Platten wurden 24 (Bakterien) bzw. 72 h (Pilze) bei 36±1 °C (Bakterien) resp. 20±1 °C (Pilze) bebrütet und ausgewertet.

Der komplette Versuch wurde in einem unabhängigen Experiment wiederholt. In allen Fällen konnten die Ergebnisse reproduziert werden.

Als MHK wurde diejenige Wirkstoffkonzentration angegeben, bei der makroskopisch kein Wachstum vorhanden war. Minimales, kaum sichtbares Wachstum oder wenige, kleine Einzelkolonien wurden nicht bewertet. Auf den wirkstofffreien Wachstumskontrollen waren alle Testkeime als "rasenförmiger", ca. 0,5 $cm^2$ großer "Fleck" gewachsen (Vorbedingung der Auswertung). Beispielsweise wurden folgende Testkeime geprüft (Dichte in KBE/ml):

| | |
|---|---|
| - Staphylococcus (S.) aureus | ATCC 6538 (1,2 x 10(6)) |
| - Pseudomonas (Ps.) aeruginosa | ATCC 27853 (1,0 x 10(6)) |
| - Escherichia (E.) coli | ATCC 8739 (1,4 x 10(6)) |
| - Candida (C.) tropicalis | DSM 4238 (0,8 x 10(6)) |
| - Aspergillus (A.) niger | ATCC 16404 (0,9 x 10(6)) |
| - Microsporum (M.) canis | CBS 38564 (1,0 x 10(6)) |
| - Trichophyton (T.) mentagrophytes | CBS 26379 (0,9 x 10(6)) |
| - T. rubrum | DSM 4167 (1,3 x 10(6)) |
| - Epidermophyton (E.) floccosum | CBS 55384 (1,4 x 10(6)) |

Tabelle 2

Ergebnisse der Agardilutionstests: MHK in µg/ml
Wachstum der Testkeime: +
kein Wachstum der Testkeime: -
K = wirkstoffreie Kontrollen

| Testkeime | K 1 | K 2 | Verbindung nach Beispiel Nr. 1 | 2 | 3 | 7 | 8 | 9 | 10 | 11 | 12 | 13 | 14 | 15 | 16 | 17 | 18 | 19 |
|---|---|---|---|---|---|---|---|---|---|---|---|---|---|---|---|---|---|---|
| S.aureus | + | + | >5,0 | >5,0 | >5,0 | >5,0 | >5,0 | >5,0 | >5,0 | >5,0 | >5,0 | >5,0 | >5,0 | >5,0 | >5,0 | >5,0 | >5,0 | >5,0 |
| Ps.aeruginosa | + | + | >5,0 | >5,0 | >5,0 | >5,0 | >5,0 | >5,0 | >5,0 | >5,0 | >5,0 | >5,0 | >5,0 | >5,0 | >5,0 | >5,0 | >5,0 | >5,0 |
| E.coli | + | + | >5,0 | >5,0 | >5,0 | >5,0 | >5,0 | >5,0 | >5,0 | >5,0 | >5,0 | >5,0 | >5,0 | >5,0 | >5,0 | >5,0 | >5,0 | >5,0 |
| C.tropicalis | + | + | 0,1 | 0,1 | 0,5 | 0,1 | 0,5 | 0,1 | 0,1 | 0,1 | 0,5 | 0,1 | 0,5 | 0,5 | 0,5 | 0,5 | 0,1 | 0,1 |
| A.niger | + | + | 0,1 | 0,1 | 0,1 | 0,1 | 0,5 | 0,5 | 0,5 | 1,0 | 0,5 | 0,1 | 0,5 | 0,5 | 0,5 | 0,5 | 1,0 | 0,5 |
| M.canis | + | + | 5,0 | 5,0 | 5,0 | 5,0 | 5,0 | 5,0 | 5,0 | 5,0 | 5,0 | 5,0 | >5,0 | 5,0 | 5,0 | 5,0 | 5,0 | 5,0 |
| T.mentagrophytes | + | + | 5,0 | 5,0 | 5,0 | 5,0 | 5,0 | 5,0 | 5,0 | 5,0 | 5,0 | 5,0 | >5,0 | 5,0 | 5,0 | 5,0 | 5,0 | 5,0 |
| T.rubrum | + | + | 5,0 | 5,0 | 5,0 | 5,0 | 5,0 | 5,0 | 5,0 | 5,0 | 5,0 | 5,0 | >5,0 | 5,0 | 5,0 | 5,0 | 5,0 | 5,0 |
| E.floccosum | + | + | 5,0 | 5,0 | 5,0 | 5,0 | 5,0 | 5,0 | 5,0 | 5,0 | 5,0 | 5,0 | >5,0 | 5,0 | 5,0 | 5,0 | 5,0 | 5,0 |

Gegenüber dem Testkeim Exophiola jeanselmei zeigen die Verbindungen der vorliegenden Erfindung ebenfalls gute Hemmeffekte.

Beispielsweise seien genannt:

| Verbindung nach Beispiel Nr. | MHK [µg/ml] |
|---|---|
| 1 | 10 |
| 3 | 30 |
| 8 | 10 |
| 10 | 10 |
| 16 | 3 |
| 17 | 3 |
| 19 | 10 |

Im Modell der Meerschweinchen-Trichophytie (Trichophyton mentagrophytes), vgl. Heffter-Heubner: Handbuch der exp. Pharmakologie, Vol. XVI/II A, sind die neuen Verbindungen bei äußerlicher Anwendung auch rezidivfrei gut wirksam.

Die Wirkungen der Prüfsubstanzen bei topischer Anwendung gegen Exophiola jennselmei als Verursacher von Subkutanmykosen und im Modell der experimentellen C. albicans vaginitis waren ebenfalls gut.

Die neuen Verbindungen sind auch oral wirksam. Im Modell der experimentellen generalisierten Candidose der Maus bzw. im Modell der experimentellen Vaginitis mit Candida albicans an der Ratte konnten mit den Prüfsubstanzen in niedrigen therapeutischen Dosen nach oraler Gabe gute Ausheilungen der Infektionen erreicht werden.

Die Verbindungen sind daher besonders zur äußerlichen, aber auch oralen Behandlung von Pilzinfektionen an Mensch und Tier geeignet. Als Indikationsgebiete an Mensch und Tier sind beispielsweise zu nennen: Subkutanmykosen und Dermatomykosen, insbesondere verursacht durch Dermatophyten, wie Spezies der Gattungen Epidermophyton, Microsporum oder Trichophyton, Hefen, wie Spezies der Gattungen Candida und Schimmelpilze, wie Spezies der Gattungen Aspergillus, Mucor oder Absidia.

Die Verbindungen können allein oder zusammen mit anderen bekannten Wirkstoffen, insbesondere Antibiotika, verwendet werden.

Die Herstellung der chemotherapeutischen Mittel oder Zubereitungen mit üblichen festen, halbfesten oder flüssigen Trägerstoffen oder Verdünnungsmitteln und den üblicherweise verwendeten pharmazeutisch-technischen Hilfsstoffen entsprechend der gewünschten Applikationsart mit einer zur Anwendung geeigneten Dosierung erfolgt in üblicher Weise, insbesondere durch Vermischen (vgl. H. Sucker et al., Pharmazeutische Technologie, Thieme-Verlag, Stuttgart, 1978).

Als Darreichungsformen kommen beispielsweise Tabletten, Dragees, Kapseln, Pillen, wäßrigen Lösungen, Suspensionen und Emulsionen, gegebenenfalls sterile injizierbare Lösungen, nicht-wäßrige Emulsionen, Suspensionen und Lösungen, Salben, Cremes, Pasten, Lotions etc. in Betracht.

Die therapeutisch wirksame Verbindung ist in pharmazeutischen Zubereitungen vorzugsweise in einer Konzentration von 0,01 bis 90 Gew.-% der Gesamtmischung vorhanden.

Im allgemeinen können bei oraler Verabfolgung sowohl in der Human- als auch in der Veterinärmedizin der oder die Wirkstoffe in Mengen von etwa 1,0 bis etwa 50,0, vorzugsweise 2 bis 10 mg/kg Körpergewicht pro Tag, vorzugsweise in Form mehrerer Einzelgaben zur Erzielung der gewünschten Ergebnisse verabreicht werden. Es kann jedoch erforderlich sein, von den genannten Dosierungen abzuweichen, und zwar in Abhängigkeit von der Art und der Schwere der Erkrankung, der Art der Zubereitung und der Applikation des Arzneimittels sowie dem Zeitraum bzw. Intervall, innerhalb welchem die Verabreichung erfolt. So kann es in einigen Fällen ausreichend sein, mit weniger als der oben genannten menge Wirkstoffe auszukommen, während in anderen Fällen die oben angeführte Wirkstoffmenge überschritten werden muß.

Beispiele für pharmazeutische Zübereitungen:

Beispiel A

Tablette mit 250 mg Wirkstoff

Zusammensetzung für 1000 Tabletten:

| | |
|---|---|
| Wirkstoff | 250 g |
| Kartoffelstärke | 100 g |
| Milchzucker | 50 g |
| Gelatinelösung 4 % | 45 g |
| Talkum | 10 g |

Herstellung:

Der fein gepulverte Wirkstoff, Kartoffelstärke und Milchzucker werden gemischt. Die Mischung wird mit ca. 45 g 4 % Gelatinelösung durchfeuchtet, feinkörnig granuliert und getrocknet. Das trockene Granulat wird gesiebt, mit 10 g Talkum vermischt und auf einer Rundläufer-Tablettiermaschine zu Tabletten verpreßt. Die Tabletten werden in dicht schließende Behälter aus Polypropylen gefüllt.

Beispiel B

Creme aus 1 % Wirkstoff

| | |
|---|---|
| Wirkstoff | 1,0 g |
| Glycerinmonostearat | 10,0 g |
| Cetylalkohol | 4,0 g |
| Polyethylenglykol-400-stearat | 10,0 g |
| Polyethylenglykol-sorbitanmonostearat | 10,0 g |
| Propylenglykol | 6,0 g |
| p-Hydroxybenzoesäuremethylester | 0,2 g |
| Entmineralisiertes Wasser ad | 100,0 g |

Herstellung:

Der feinst gepulverte Wirkstoff wird mit Propylenglykol suspendiert und die Suspension in die auf 65 °C erwärmte Schmelze aus Glycerinmonostearat, Cetylalkohol, Polyethylenglykol-400-stearat und Polyethylenglykolsorbitanmonostearat gerührt. In diese Mischung wird die 70 °C heiße Lösung des p-Hydroxybenzoesäuremethylesters in Wasser emulgiert. Nach dem Erkalten wird die Creme über eine Kolloidmühle homogenisiert und in Tuben abgefüllt.

Beispiel C

Puder mit 1 % Wirkstoff

| | |
|---|---|
| Wirkstoff | 1,0 g |
| Zinkoxid | 10,0 g |
| Magnesiumoxid | 10,0 g |
| Hochdisperses Siliciumdioxid | 2,5 g |
| Magnesiumstearat | 1,0 g |
| Talkum | 75,5 g |

Herstellung:

Der Wirkstoff wird auf einer Luftstrahlmühle mikronisiert und mit den anderen Bestandteilen homogen vermischt. Die Mischung wird durch ein Sieb (Maschenweite Nr. 7) geschlagen und in Polyethylenbehälter mit Streueinsatz abgefüllt.

## Patentansprüche

1. Antimykotische Mittel, enthaltend eine Verbindung der Formel

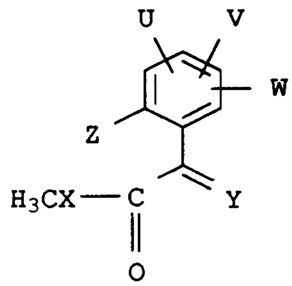

1

in der

| | |
|---|---|
| =Y | $-CH-OCH_3$, $=CH-CH_3$, $=CH-CH_2-CH_3$, $=CH-SCH_3$ oder $=N-OCH_3$ bedeutet, |
| X | ein Sauerstoffatom oder - falls Y eine $=N-OCH_3$-Gruppe ist - auch eine NH-Gruppe bedeutet, |
| Z | Halogen (F, Cl, Br, J), Nitro, Cyano, gegebenenfalls substituiertes Alkyl, gegebenenfalls substituiertes Cycloalkyl, gegebenenfalls substituiertes Aralkyl, gegebenenfalls substituiertes Aryloxyalkyl, gegebenenfalls substituiertes Arylthioalkyl, gegebenenfalls substituiertes Heteroarylalkyl, gegebenenfalls substituiertes Heteroaryloxyalkyl, gegebenenfalls substituiertes Heteroarylthioalkyl, gegebenenfalls sübstituiertes Alkenyl, gegebenenfalls substituiertes Aralkenyl, gegebenenfalls substituiertes Aryloxyalkenyl, gegebenenfalls substituiertes Arylthioalkenyl, gegebenenfalls substituiertes Heteroarylalkenyl, gegebenenfalls substituiertes Heteroaryloxyalkenyl, gegebenenfalls substituiertes Heteroarylthioalkenyl, gegebenenfalls substituiertes Alkinyl, gegebenenfalls substituiertes Aryl, gegebenenfalls substituiertes Heteroaryl, gegebenenfalls substituiertes Amino, gegebenenfalls substituiertes Arylazo, gegebenenfalls substituiertes Acylamino, $OR^{12}$, $SR^{13}$, $SOR^{14}$, $SO_2R^{15}$, $-COOR^{16}$, $-CONR^{17}R^{18}$, $-COR^{19}$, $-CR^{20}=NR^{21}$, $-N=CR^{22}R^{23}$, $-CR^{24}=N-OR^{25}$, $-CR^{25}R^{26}-O-N=CR^{27}R^{28}$ bedeutet und |
| U, V, W | gegebenenfalls gleich oder verschieden sind und Wasserstoff bedeuten oder eine der für Z genannten Bedeutungen haben können, oder in der |

zwei der Gruppierungen Z, U, V oder W in benachbarten Positionen des Phenylrings gegebenenfalls zusammen einen gegebenenfalls substituierten, an den Phenylring ankondensierten fünf-oder sechsgliedrigen, aromatischen oder aliphatischen Ring bilden, der ggf. ein bis drei Heteroatome (N, S, O) enthalten kann, und

die Gruppierungen $R^{12}$ bis $R^{28}$ gegebenenfalls gleich oder verschieden sind und Wasserstoff, gegebenenfalls substituiertes $C_1$-$C_8$-Alkyl, gegebenenfalls substituiertes $C_2$-$C_8$-Alkenyl, gegebenenfalls substituiertes $C_2$-$C_8$-Alkinyl, gegebenenfalls substituiertes Cycloalkyl, gegebenenfalls substituiertes Cycloalkylalkyl, gegebenenfalls substituiertes Aryl, gegebenenfalls substituiertes Heteroaryl, gegebenenfalls substituiertes Aralkyl, gegebenenfalls substituiertes Heteroarylalkyl, gegebenenfalls substituiertes Aryloxyalkyl, gegebenenfalls substituiertes Arylthioalkyl, gegebenenfalls substituiertes Heteroaryloxyalkyl oder gegebenenfalls substituiertes Heteroarylthioalkyl bedeuten.

2. Antimykotische Mittel, enthaltend eine Verbindung der Formel 1 gemäß Anspruch 1, in der U, V und W gleich oder verschieden sind und Wasserstoff, Fluor, Chlor, Brom, Iod, Cyano, Nitro, Trifluormethyl, Methyl oder Methoxy bedeuten.

3. Antimykotische Mittel, enthaltend eine Verbindung der Formel 1 gemäß Anspruch 1, in der U, V und W Wasserstoff bedeuten.

4. Antimykotische Mittel, enthaltend eine Verbindung der Formel 1 gemäß Anspruch 1, in der Z für die

Gruppe $OR^{12}$ oder $SR^{13}$ steht und $R^{12}$ und $R^{13}$ die im Anspruch 1 angegebenen Bedeutungen haben.

5. Antimykotische Mittel, enthaltend eine Verbindung der Formel 1 gemäß Anspruch 1, in der $R^{12}$ und $R^{13}$ gegebenenfalls substituiertes Alkyl, gegebenenfalls substituiertes Aryl, oder gegebenenfalls substituiertes fünf- oder sechsgliedriges Heteroaryl mit 1 bis 3 Heteroatomen (N, O, S) bedeuten.

6. Antimykotische Mittel, enthaltend eine Verbindung der Formel

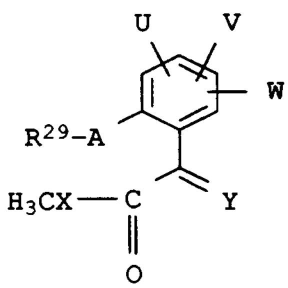

1a

in der U, V, W, X und Y die im Anspruch 1 angegebene Bedeutung haben und -A- für die Gruppierungen $-CR^{30}=CR^{31}-$, $-CHR^{30}-CHR^{31}-$, $-O-CHR^{30}-$, $-CHR^{30}-O-$, $-CHR^{30}-S-$, $-S-CHR^{30}-$, $O-N=CR^{30}-$ oder $-CHR^{30}-$steht und in der $R^{29}$ gegebenenfalls substituiertes Alkyl, gegebenenfalls substituiertes Alkenyl, gegebenenfalls substituiertes Aryl, gegebenenfalls substituiertes Heteroaryl, gegebenenfalls substituiertes Aralkyl, gegebenenfalls substituiertes Heteroarylalkyl bedeuten und $R^{30}$ und $R^{31}$ gleich oder verschieden sind und Wasserstoff oder geradkettiges oder verzweigtes $C_1$-$C_4$-Alkyl bedeuten.

7. Antimykotische Mittel, enthaltend eine Verbindung der Formel 1a gemäß Anspruch 6, in der die Gruppe $R^{29}$-A- für die Gruppierung $R^{32}R^{33}C=N-O-CHR^{31}-$ steht, in der $R^{31}$ die im Anspruch 5 genannten Bedeutungen hat und $R^{32}$ und $R^{33}$ unabhängig voneinander Wasserstoff, $C_1$-$C_{12}$-Alkyl, $C_1$-$C_4$-Halogenalkyl, $C_1$-$C_4$-Alkoxy-$C_1$-$C_4$-alkyl, $C_1$-$C_4$-Alkylthio-$C_1$-$C_4$-alkyl, Aryl-$C_1$-$C_4$-alkyl, Aryloxy-$C_1$-$C_4$-alkyl, Arylthio-$C_1$-$C_4$-alkyl, Heteroaryl-$C_1$-$C_4$-alkyl, $C_2$-$C_{12}$-Alkenyl, Aryl-$C_2$-$C_4$-alkenyl bedeuten, Heteroaryl-$C_2$-$C_4$-alkenyl, $C_2$-$C_{12}$-Alkinyl, $C_3$-$C_6$-Cycloalkyl, Aryl, Heteroaryl, Cyano oder eine der Gruppen (a) bis (d)

$COOR^{34}$ (a) $CONR^{35}R^{36}$ (b)

$COR^{37}$ (c) $CR^{38}=NOR^{39}$ (d) bedeuten

oder $R^{32}$ und $R^{33}$ zusammen mit dem Kohlenstoffatom, an das sie gebunden sind, einen 4- bis 7-gliedrigen, gegebenenfalls ein Sauerstoff- oder Schwefelatom enthaltenden Ring, welch letzterer einen oder zwei ankondensierte aromatische Ringe, z.B. gegebenenfalls substituierte Benzolringe enthalten kann, bedeuten
und $R^{34}$, $R^{35}$, $R^{36}$, $R^{37}$, $R^{38}$ und $R^{39}$ jeweils Wasserstoff, $C_1$-$C_4$-Alkyl, Aryl oder Heteroaryl bedeuten.

8. Antimykotische Mittel, enthaltend eine Verbindung der Formel 1a gemäß Anspruch 6, in denen $R^{29}$ für fünf- oder sechsgliedriges, gegebenenfalls ein- oder mehrfach durch Halogen, $C_1$-$C_{12}$-Alkyl, $C_1$-$C_{12}$-Halogenalkyl, $C_2$-$C_{12}$-Alkenyl, $C_2$-$C_{12}$-Alkinyl, gegebenenfalls substituiertes Aryl, gegebenenfalls substituiertes Heteroaryl, gegebenenfalls substituiertes Aralkyl, $C_1$-$C_{12}$-Alkoxy, $C_1$-$C_{12}$-Halogenalkoxy, $C_2$-$C_{12}$-Alkenyloxy, $C_2$-$C_{12}$-Alkinyloxy, gegebenenfalls substituiertes Aryloxy, Formyl, $C_1$-$C_{12}$-Acyl, Cyano, Trifluormethyl, Nitro oder mit der Gruppe $-CR^{34}=N-OR^{35}$ substituiertes und/oder gegebenenfalls mit einem Benzolring anelliertes Aryl, Benzyl oder Heteroaryl bedeutet, wobei $R^{34}$ für Wasserstoff oder $C_1$-$C_4$-Alkyl und $R^{35}$ für Wasserstoff, $C_1$-$C_8$-Alkyl, $C_2$-$C_8$-Alkenyl oder $C_2$-$C_8$-Alkinyl steht.

9. Verfahren zur Bekämpfung von Mykosen, wobei eine Verbindung der Formel 1 oder 1a gemäß Anspruch 1 oder 6 als Wirkstoff eingesetzt wird.

10. Verfahren zur Herstellung eines Mittels zur Bekämpfung von Mykosen, wobei eine Verbindung der

Formel 1 oder 1a gemäß Anspruch 1 oder 6 als Wirkstoff neben üblichen galenischen Hilfsmitteln eingesetzt wird.

Europäisches Patentamt

# EUROPÄISCHER RECHERCHENBERICHT

Nummer der Anmeldung

EP 92 10 8035

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,X | EP-A-0 337 211 (BASF AKTIENGESELLSCHAFT) 18. Oktober 1989<br>* Seite 33, Zeile 50 - Seite 33, Zeile 51; Ansprüche 1,2 * | 1-3,6,10 | A61K31/00<br>A61K31/165<br>A61K31/33<br>A61K31/215 |
| D,Y | EP-A-0 242 070 (IMPERIAL CHEMICAL INDUSTRIES PLC ) 21. Oktober 1987<br>* Seite 27 - Seite 28; Anspruch 1 * | 1-8,10 | |
| D,Y | EP-A-0 426 460 (UBE INDUSTRIES, LTD) 8. Mai 1991<br>* Seite 7, Zeile 45 - Zeile 48; Anspruch 1 * | 1-8,10 | |
| D,Y | EP-A-0 254 426 (IMPERIAL CHEMICAL INDUSTRIES PLC ) 27. Januar 1988<br>* Seite 20, Zeile 24 - Zeile 52; Anspruch 1 * | 1-8,10 | |
| Y | US-A-3 826 836 (KARL HEINZ BUCHEL ET AL.) 30. Juli 1974<br>* Zusammenfassung * | 1-8,10 | |
| | ----- | | **RECHERCHIERTE SACHGEBIETE (Int. Cl.5 )** |
| | | | A61K |

Der vorliegende Recherchenbericht wurde für alle Patentansprüche erstellt

| Recherchenort | Abschlußdatum der Recherche | Prüfer |
|---|---|---|
| MUENCHEN | 11 AUGUST 1992 | TZSCHOPPE D.A. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)